# EUROPEAN PATENT SPECIFICATION

(11) **EP 1 585 821 B1**
(45) Date of publication and mention of the grant of the patent: **05.11.2008**
(21) Application number: 03754748.6
(22) Date of filing: 22.09.2003
(51) Int. Cl.: C12P 7/62

(54) **BIOCONVERSION OF XYLAN AND LEVULINIC ACID TO BIODEGRADABLE THERMOPLASTICS**
BIOKONVERSION VON XYLAN UND LEVULINSäURE ZU BIOLOGISCH ABBAUBAREN THERMOPLASTEN
BIOCONVERSION DE XYLANE ET D'ACIDE LEVULIQUE EN THERMOPLASTIQUES BIODEGRADABLES

(30) Priority: 23.09.2002 US 412719 P
(43) Date of publication of application: 19.10.2005
(73) Proprietor: THE RESEARCH FOUNDATION OF STATE UNIVERSITY OF NEW YORK, Albany, New York 12201-0009 (US)
(72) Inventor: NAKAS, James, P., Lafavette, NY 13084 (US); TANENBAUM, Stuart, W., Manlius, NY 13104 (US); KEENAN, Thomas, Rochester, NY 14616 (US)
(74) Representative: Schurack, Eduard F.
(86) International application number: PCT/US2003/029464
(87) International publication number: WO 2004/027076

(56) References cited:
- BOZELL J J ET AL: "Production of levulinic acid and use as a platform chemical for derived products" RESOURCES CONSERVATION AND RECYCLING, ELSEVIER SCIENCE PUBLISHER, AMSTERDAM, NL, vol. 28, no. 3-4, February 2000 (2000-02), pages 227-239, XP004185669 ISSN: 0921-3449
- PAGE W.J.: 'Waste sources for polyhydroxyalkanoate production' INT'L SYMPOSIUM ON BACTERIAL POLYHYDROXYALKANOATES 1997, pages 56 - 65, XP008045637
- RAMSEY J.M. ET AL.: 'Hemicellulose as a potential substrate for production of poly-beta-hydroxyalkanoates' CAN J. MICROBIOL. vol. 41, no. SUPPL.1, 1995, pages 262 - 266, XP008045635
- BERTRAND J.L. ET AL.: 'Biosynthesis of poly-beta-hydroxyalkanoates from pentosesby pseudomonas pseudoflava' APPLIED ENVIRON. MICROBIOL. vol. 56, no. 10, October 1990, pages 3133 - 3138, XP008045636
- LEE S.Y.: 'Poly(3-hydroxybutyrate) production from xylose by recombinant Escherichia coli' BIOPROCESS AND BIOSYSTEMS ENGINEERING vol. 18, May 1998, pages 397 - 399, XP002985876
- CHUNG S.H. ET AL.: 'Effect of levulinic acid on the production of poly(3-hydroxybutyrate-co-3-hydroxyvalerate ) by ralstonia eutropha KHB-8862' J. MICROBIOL. vol. 39, no. 1, March 2001, pages 79 - 82, XP008045625
- JANG J.H ET AL.: 'Effect of levulinic acid on cell growth and poly-beta-hydroxyalkanoate production by alcaligenes sp. SH-69' BIOTECHNOL. LETT. vol. 18, no. 2, 1996, pages 219 - 224, XP001022409
- MCMILLAN J.D.: 'Conversion of hemicellulose hydrolyzates to ethanol' ACS SYMPOSIUM SERIES vol. 566, 1994, pages 411 - 437, XP008045627
- PARAJO J.C. ET AL.: 'Biotechnological production of Xylitol. Part 3: operation in culture media made from lignocellulose hydrosylates' BIORESOURCE TECHNOLOGY vol. 66, 1998, pages 25 - 40, XP002985877
- EKEN-SARACOGLU N. ET AL.: 'Comparison of different pretreatments in Ethanol fermentation using corn cob hemicellulosic hydrosylkate with pichia stipitis and Candida shehatae' BIOTECHNOL. LETT. vol. 22, 2000, pages 855 - 858, XP002985878
- MUSSATTO S.I. ET AL.: 'Hydrolysate detoxification with activated charcoal for Xylitol production by Candida guilliermondii' BIOTECHNOL. LETT. vol. 23, 2001, pages 1681 - 1684, XP002985879
- MARTINEZ A. ET AL.: 'Detoxification of dilute acid hydrolysates of lignocellulose with lime' BIOTECHNOL. PROG. vol. 17, 2001, pages 287 - 293, XP002985880

## Description

### BACKGROUND OF THE INVENTION

Polyhydroxyalkanoates (PHAs) are biodegradable polyesters that in some microgorganims are an intracellular carbon and energy reserve synthesized by a variety of microorganisms when carbon sources are provided in excess and growth is impaired by the lack of at least one other nutrient. Recently, PHAs have received increased attention because of their thermoplastic or elastomeric properties that resemble those of petroleum-based plastics, yet are completely biodegradable in the environment (Holmes 1988). Thus, in addition to being synthesized biologically, these alternative polymeric materials are capable of being converted to the harmless degradation products of CO₂ and H₂O through natural microbiological degradation (Imam *et al*. 1999). With the appropriate choice of microorganism, carbon source, co-substrate, and culture conditions, a biodegradable copolyester can be produced with properties similar to those of polypropylene, while avoiding many of the environmentally recalcitrant characteristics of petroleum-based plastics (Bertrand *et al*. 1990). In addition to being biodegradable, PHA polyesters are also recyclable, similar to the petrochemical-derived thermoplastics (Madison and Huisman 1999). Since the vast majority of plastics are synthesized from petroleum-based feedstocks, our approach to enhance this technology will focus on the use of paper industry residue, namely xylan, as the major carbon source for microbial conversion to these environmentally compatible polymers.

This new generation of biopolymer thermoplastics represents an attractive alternative to plastics derived from fossil fuel-based feedstocks during a time of rising oil prices, waste management problems, and continued global pollution. Another marketable advantage of these biopolymers is their production from renewable resources used as the primary carbon source and co-substrate. Production based on relatively inexpensive substrates could make PHA-derived thermoplastics more economically competitive with petroleum-based plastics, as the major costs in PHA production are the substrate and the separation process (Byrom 1987). Ramsay *et al*. (1995) demonstrated the ability of *Pseudomonas pseudoflava* to produce poly-β-hydroxyalkanoates using the major sugars present in hemicellulose as sole carbon sources. Naylor et al., U.S. 5,871,980, disclose production of PHA by fermentation of *Alcaligenes sp.* by feeding the cells an aliphatic acid typically containing one or more alkyl groups containing 8-25 carbon atoms. Naylor et al. demonstrate that optional addition of an odd-number carbon molecule, e.g., propionic acid or n-propyl alcohol, can result in the production of PHAs containing up to 30 mol% valerate.

Poly-3-hydroxybutyrate [P(3HB)] is probably the best characterized of all the PHAs. However, polymers of [P(3HB)] are highly crystalline and brittle, resulting in a rather limited range of applications. Because of these limitations, recent investigations have focused on the synthesis of a co-polymer consisting of 3-hydroxybutyrate and 3-hydroxyvalerate to create poly(3-hydroxybutyrate-co-3-hydroxyvalerate) [P(3HB-co-3HV)], a polyester with increased strength and more flexible mechanical properties conferred by its unique monomeric composition (Holmes 1988). The physical and thermal properties of the [P(3HB-co-3HV)] co-polymer and other related PHA polyesters can be regulated by varying their molecular structures and monomeric compositions through judicious choice of microorganism, substrate/co-substrate ratios, and general fermentation conditions. The family of PHA thermoplastics produced by our research encompasses a wide range of industrially useful polymeric materials. This class of polymers displays physical and mechanical properties ranging from hard crystalline plastics to elastic rubbers, with melting temperature profiles that allow for commercial extrusion, injection molding, and fiber spinning to create a variety of value-added products (Sudesh *et al*. 2000).

The co-polymer, [P(3HB-co-3HV)], can be synthesized by several strains of bacteria, including *Ralstonia eutropha* (formerly *Alcaligenes eutrophus*), *Methylobacterium sp*., select species of *Pseudomonas*, and various recombinant clones growing on mixtures of the appropriate monomer-supplying carbohydrate and an odd-chain fatty acid, such as propionic acid, provided as a co-substrate with the primary carbon source (Madison and Huisman 1999).

Recombinantly engineered bacterial strains can be manipulated to modulate the HB:HV ratio in the co-polymer. For example, Aldor and Keasling (2001) demonstrated that the composition of [P(3HB-co-3HV)] can also be controlled by metabolic engineering of a recombinant *Salmonella enterica* strain, which involved "dialing the composition" by varying the induction level of a critical PHA biosynthetic gene. Schubert *et al*. (1998) report PHA expression in *E*. *coli* transformed with constitutively expressed PHA biosynthesis genes of R. *eutropha*. Choi *et al*. (1998) report cloning of PHA biosynthesis genes from *Alcaligenes latus*. See, also, *e*.*g*., U.S. 6,593,116 and 6,316,262.

Levulinic acid is a 4-keto-pentanoic acid obtainable via acid hydrolysis of 6-carbon sugars, which can be derived from carbohydrate-containing renewable wastestream residues (Bozell *et al*. 2000). Co-polymers of [P(3HB-co-3HV)] have been produced microbially (*Alcaligenes sp*. SH-69) from glucose and levulinic acid, with this organic acid co-substrate displaying a significant stimulatory effect on both cell growth and co-polymer accumulation (Jang and Rogers 1996). Jang and Rogers (1996) report that levulinic acid is an inexpensive substrate that compares favorably with propionic, valeric, or pentanoic acids as a co-substrate for PHA production. Steinbuchel *et al*. (1998) describe the production and characterization of polyesters containing 4-hydroxyvaleric acid and medium-chain length hydroxyalkanoic acids from octanoic acid as a principal carbon source and levulinic acid as a co-substrate.

Chung *et al*. (2001) suggest repeated additions of equal amounts of levulinic acid as a secondary carbon source to a medium containing *Ralstonia eutropha* and glucose, sucrose or sorbitol as a primary carbon source. This way, the concentration of levulinic acid could be kept at low levels compared with experiments in which levulinic acid was added once at the beginning of cell culture, thereby reducing its toxic and inhibitory effects on cell growth. However, while enhancing overall cell mass and production of [P(3HB-co-3HV)] copolyesters, the final 3HV mol% in the copolyesters is decreased significantly.

### SUMMARY OF THE INVENTION

The invention comprises the use of xylose as a primary carbon source, and levulinic acid as a secondary carbon source, or co-substrate, for microbial fermentation using *Burkholderia cepacia* as microorganism capable of converting carbon to PHA co-polymer comprising 3-OH-valeryl (3-HV) and 3-OH-butyryl (3-HB) monomers. According to the invention, the 3HV mol% is increased by adding a second quantity of levulinic acid to the medium between about 16 hours and about 24 hours after the first quantity of levulinic acid was added, wherein the second quantity of levulinic acid is greater than the first quantity of levulinic acid.

In another aspect of the invention, the relative amounts of hydroxyvalerate (HV) and hydroxybutyrate (HB) in the PHA are modulated by adjusting the ratio of xylose to the co-substrate levulinic acid.

In another aspect of the invention, oxidized or otherwise derivatized xylose, levulinic acid, or both are employed to produce polymers with similarly derivatized monomers.

### DETAILED DESCRIPTION OF THE INVENTION

One of the major limitations to the commercialization of PHAs as replacements for petroleum-based polymers remains their relatively high cost of production. For this reason, much effort has been directed toward the development of higher yielding strains and more efficient fermentations. We have utilized xylose and xylose oligomers, derived from the acid-hydrolyzed xylan component of forest biomass generated by the paper industry, as the primary carbon source. Currently the major portion of this eminently fermentable carbon source is either discharged into the environment or burned to generate heat within the plant. Willow tree biomass is a useful source of xylans because it is fast growing and because the pulping or delignification process leaves relatively large amounts of cellulose and residual xylan.

In the practice of the current invention, the ratio of the co-substrate, levulinic acid, to the primary substrate, xylose, allows for control of the 3HB/3HV composition of the co-polymer. The ratio of valerate/butyrate in the [P(3HB-co-3HV)] polymer has an industrially important influence on its physical and mechanical properties, including melting and glass transition temperatures, tensile strength, elasticity, flexural modulus, and decomposition profile (Doi 1990). The approach described herein produces a composition-regulated [P(3HB-co-3HV)] co-polymer, preferably using hemicellulosic wastestreams as the principal carbon source and appropriate formulations of levulinic acid, which also are preferably derived from inexpensive, renewable feedstocks. Preliminary experiments, using levulinic acid, indicate select microbial utilization of this co-substrate in the production of [P(3HB-co-3HV)] co-polymers, as evidenced by the progressive increase in the 3-hydroxyvaleric acid fraction, regulated by defined co-substrate additions. See, Example 2, below.

The current invention also allows for the production of polymers with advantageous properties, *e*.*g*., controlled and improved HV:HB ratios, greater viscosity, indicating higher molecular weight/longer average chain length, *etc*..

In the practice of the invention, *Burkholderia cepacia* microorganisms are cultured in accordance with standard techniques. Similarly, the PHA so produced can be harvested using standard techniques. See, *e*.*g*., Horowitz et al., U.S. 5,871,980.

The suggested process can also be used to produce derivatized PHA homopolymers, co-polymers, terpolymers, etc., e.g., by selecting fermenting microorganisms equipped with the necessary metabolic machinery for utilization of the appropriate precursor PHA substrates. This can be accomplished by genetically engineering microorganisms to express required metabolic genes, or by pre-fermentative enzymatic treatment of PHA substrates for the production of novel polymers with unique backbone and side-chain structures. The resultant polymers display unique physical, chemical, and mechanical properties, related to their monomeric compositions and crystalline structures. As noted above, methods for genetically engineering PHA-producing microorganisms are known. Techniques for pre-fermentative treatment of substrates are also within the skill of the art. For instance, xylose can be used in a PHA fermentation in conjunction with an oxidized/derivatized version of levulinic acid or other related condensation product of levulinic acid.

The hydroxyl-containing functionalities of the unpolymerized 3- hydroxybutyrate and 3- hydroxyvalerate monomers are potentially oxidizable substrates that could be condensed with other moieties to produce derivatized monomers and thus drastically different crystalline lattice structures in the resultant PHA polymers (changing physical, chemical, and mechanical properties).

The oxidation or derivatization reactions would be catalyzed by a selected enzyme prior to addition to the fermentation medium. Typically, the oxidation or condensation (e.g. to another molecule that offers unique structural features) would occur at the 3-OH position. The derivatized levulinic acid would be added to the PHA fermentation as the co-substrate with xylose, to then be recognized and acted upon by native or recombinant monomer-supplying enzymes and PHA polymerase enzymes. For example, oxidation at the 3-hydroxy position of the PHA precursor, 3-OH-valeryl-CoA, can produce unique monomers that would be recognized by the host strain's PHA synthase/polymerase and incorporated into the growing PHA chain(s). Derivatized forms of xylose can similarly be used to prepare different monomers. Thus, this invention, in some embodiments, encompasses use of oxidized or otherwise derivatized levulinic acid, xylose, or both. Such derivatized carbon sources would typically be prepared outside the fermentation and added to the fermentation medium, although genetic engineering techniques would permit the introduction into the fermenting microorganism of genes necessary to derivatize the carbon source biologically and *in situ.*

The suggested process can also be used to produce PHAs with 4-HV monomers. Steinbuchel *et al*. (1998) demonstrated the production of PHA polyesters composed of 3-HB, 3-HV, 4-HV, and medium-chain length hydroxyalkanoate monomers (hexanoate and octanoate), utilizing a recombinant strain of *Pseudomonas putida*. The controlled fermentation used octanoic and levulinic acids as carbon sources. This study purposely used levulinic acid as a precursor to 4-HV, because 4-hydroxyvaleric acid is not commercially available. In the Examples described hereinbelow, levulinic acid was the precursor to 3-HV monomers. However, by proper selection of the fermenting microorganism, e.g., by genetic engineering, or by appropriate selection of fermentation conditions, the suggested process can be used to produce polymers comprising 4-HV monomers through use of xylose and levulinic acid as the carbon sources.

If fatty acid substrates longer than levulinic acid are added to the fermentation medium, polymers that comprise medium-chain length PHA (MCL-PHA) monomers in addition to the 3-HV and 3-HB monomers can be produced. Such MCL-PHA monomers include, e.g., 3-hydroxyhexanoate (3-HHx) and 3-hydroxyoctanoate (3-HO). Fatty acids that are included as additional carbon sources include, e.g., dodecanoic acid, decanoic acid and octanoic acid, as well as longer chain fatty acids such as oleic acid or oleate or palm oil. Inclusion of such additional carbon sources can result in novel polymers with more desirable physical/mechanical properties (*e.g*. lower melting points, greater flexibility, greater elongation to break percentages, etc.).

The alkyl side chains of PHA monomers are generally saturated, although aromatic, unsaturated, halogenated, and branched functionalities have been described in the literature.

In a typical PHA production, *Burkholderia cepacia* microorganisms are cultured in a medium that utilizes xylose and levulinic acid as the principal carbon source and organic acid co-substrate, respectively. Sterilized aliquots of these substrates are added to a defined mineral salts medium, followed by inoculation. Typically, xylose is added in an amount of up to about 4% w/v final broth concentration, e.g., about 2.0 to about 3.0% w/v. Granular xylose is dissolved in distilled water, autoclaved, and added as a single dose just prior to inoculation. The 2-3 % w/v concentration of xylose appears to result in rather high cell densities and PHA product yield, with the concentration of levulinic acid being varied in order to modulate the hydroxyvalerate fraction of the copolymer.

Depending on the woody biomass source and hydrolytic procedure/conditions, the concentration of xylose estimated in the final, detoxified xylan hydrolysate typically ranges from about 1.0 to about 2.5 % w/v. Additionally, the dose and administration schedule will vary when the PHA production scheme is converted to a fermenter-based culture, in which xylose and levulinic acid may be added periodically throughout the fermentation. This type of fermentation may also be divided into two stages, the first of which provides for balanced growth conditions and relatively high substrate concentrations in order to maximize cell densities. These cells are then transferred to a nitrogen limited/unbalanced growth medium for the production of PHA polymer product.

In accordance with this invention, the xylose is preferably derived from woody biomass. Woody biomass, e.g., forest biomass, comprises three readily separable fractions: a lignin fraction, a cellulosic fraction and a hemicullulosic fraction. The hemicelullose-enriched fraction comprises xylans from which xylose can be derived. Hemicellulosic fractions of woody biomass can contain microbial growth-inhibiting substances which are preferably removed prior to use of xylose obtained therefrom. Thus, a process for detoxifying xylans is disclosed, thereby making a composition comprising the hemicellulosic fraction of woody biomass suitable for use as a fermentation carbon source. This process comprises removing volatile solvents from a hemicellulose-enriched fraction of a woody biomass, preferably by distillation under vacuum, although other methods can be used, e.g., extraction using other organic solvents such as, e.g., diethyl ether. Thereafter, the process preferably comprises adjusting the pH of the distillate to above neutral, e.g., about 8 to about 12, preferably about 10 and maintaining the high pH for several minutes to several hours, e.g., at least about 30 minutes, preferably for about 1 hr to remove volatile solvents; adjusting the pH to below neutral, e.g., about 6 to about 2, preferably about 5 to about 6, e.g., with an organic acid such as sulfuric acid; contacting the acidified preparation with a molecular sieve, e.g., activated charcoal for several minutes to several hours, e.g., at least about 30 minutes, preferably for about 1 hr; and then adjusting the pH to about 7; removing calcium, e.g., by precipitation with potassium phosphate. Optionally, this preparation is filter sterilized prior to inoculation. Also, optionally the composition is supplemented with nutritional supplements to enhance microbial growth, e.g., with salts, trace elements and vitamins, e.g., prior to filter sterilization.

A typical such procedure is as follows.
1. Using a flash evaporator, reducing a hemicellulosic fraction of a woody biomass to half volume.
2. Replacement of volume with water and flash evaporate again to half volume but do not replace volume this time.
3. Filtration through filter paper & Buchner funnel.
4. Overliming using calcium hydroxide to pH 10 and maintain for 1 hour
5. Filtration again as in #3.
6. Adjusting pH to 5.5 using sulfuric acid/add sodium sulfite (1g/L).
7. Treating with activated charcoal for 1 hour followed by filtration again as in #3.
8. Checking pH again; adjust to 7.
9. Adding potassium phosphate (monobasic) until precipitation and/or suspension formation stops (to remove the calcium).
10. Filtration to remove calcium phosphate.
11. Adding salts, trace elements and vitamins as needed for microbial growth.
12. Filter sterilization. At this point the hydrolysate is typically a clear and slightly yellow liquid and is ready for inoculation.

Various methods can be used to prepare the xylan-containing starting material, i.e., the hemicellulose-enriched fraction of woody biomass. One illustrative process, is the protocol developed by the National Renewable Energy Research Laboratory (Golden, CO), which is a procedure known as the Clean Fractionation (CF) or NREL CF process. This process is disclosed in U.S. 5,730,837. The hemicellulosic fraction produced in this process is rich in five-carbon sugars (principally xylose) and is thus termed a "C5 stream". Briefly, the procedure involves treatment of a woody biomass with organic solvents (namely, methyl isobutyl ketone (MIBK) and ethanol (EtOH)), an acid, e.g., sulfuric acid, and water for selective separation of the three principal woody biomass components: lignin, cellulose, and hemiceullose. The hemicellulosic stream is a dilute aqueous solution that contains growth inhibiting concentrations of MIBK and EtOH, for which the distillation/removal steps (#2 and #3, above) are included in the hydrolysis-detoxification procedure described above.

In a second illustrative method, wood chips from a variety of tree species are treated at high temperatures (140-180°C) and pressures in stainless steel chambers, using water as an extracting solvent. In this process, the lignin framework is partially degraded, allowing for a rather high degree of hemicellulosic extraction into the aqueous phase. The remaining wood chips are saved for other cellulosic applications and the opaque, muddy-brown xylan hydrolysate is detoxified, e.g., by acid hydrolysis as described above, for subsequent PHA fermentation. The distillation steps (#2 and #3, above) are not necessary for this water-based hydrolysate, although the remainder of the detoxification procedure is useful.

Levulinic acid can be obtained, for example, from the Biofine Corporation (Glens Falls, New York) as a refined, crystalline substance. The Biofine process is basically a two-stage, acid hydrolysis of 6-carbon sugars that results in the cost-effective production of levulinic acid in relatively high yield. The process is described in a paper published by Bozell et al. (2000). Briefly, carbohydrate-containing materials are hydrolyzed in a first reactor at 210-230°C in the presence of 1-5% mineral acid. The hydroxymethylfurfural produced in this initial hydrolysis is removed and continuously supplied to a second reactor for further hydrolysis at 195-215°C to produce levulinic acid. The levulinic acid yield is on the order of 60 % or greater.

Levulinic acid is generally added to the PHA fermentation medium first as a low initial dose (approx. 0.07 % w/v) together with xylose. Then, after about 16 to about 24 hours, when PHA polymer formation is estimated to begin (optical density at 540 nm, indicating cell density, at this point is generally 0.3-0.5 using *B*. *cepacia* in a xylose/levulinic acid PHA fermentation), a second dose of levulinic acid is added, wherein the second quantity of levulinic acid is greater than the first quantity to increase the mol% of 3HV in the resulting PHA. The important aspect of levulinic acid addition at this time is that PHA production is just beginning, as the nitrogen concentration in the shake-flask has declined to levels insufficient for optimal growth and thus conducive to PHA accumulation. Using this shake-flask fermentation procedure, formation of the poly(hydroxybutyrate) homopolymer is minimized and production of the desired [P(3HB-co-3HV)] copolymer with a high mol% of 3HV is encouraged. Levulinic acid is added at various concentrations in the second dose (*i*.*e*. generally concentrations ranging up to 0.8 % w/v), depending on the molar fraction of hydroxyvalerate desired in the P(3HB-co-3HV) copolymer.

As disclosed above, typically, levulinic acid is added as a 0.07 % w/v initial dose. To prepare PHAs with increasing amounts of 3-HV monomer, the acid is then added in progressively increasing second doses (generally 0.1 to 0.8 % w/v) at about 20 hours post-inoculation, depending on the desired molar fraction of HV in the resultant PHA. Assuming 2.2 % w/v xylose, the ratio of levulinic acid to xylose in the final fermentation medium thus ranges to 0.4, i.e., 0.87/2.2 (second levulinic acid dose of 0.8 % w/v). Levulinic acid is a known inhibitor of tetrapyrrole biosynthesis and can exert growth and PHA-inhibiting effects when administered at sufficiently high concentrations. For this reason, the concentration of levulinic acid added to the cultures is often limited in order to obtain quantities of the PHA product sufficient for isolation and characterization.

After culturing the microorganisms for up to about a week, the PHA is collected, e.g., by freeze-drying cells collected from the culture broth, grinding the dried cells, suspending the ground cells in an organic solvent, and then precipitating the PHA, e.g., with ethanol.
The precipitate can be filtered and re-precipitated to improve purity prior to final drying.

The PHA-laden shake-flask cultures are generally harvested by centrifugation about 65 to about 75 hours post-inoculation (assuming *B*. *cepacia-based* fermentation of xylose and levulinic acid). With shake-flask cultures of *B*. *cepacia*, the OD540 of the broth (optical density at 540 nm) ranges from 2.5-3.5 at this time. The point of optimizing harvest time is to maximize product yield in terms of percent dry biomass occupied by the PHA polymers. If the fermentation is allowed to proceed longer than this optimal period of time, then the microrganism will begin to metabolize the polymer as a carbon and energy reserve (*i.e* when available exogenous carbon sources in the fermentation medium have been exhausted). Thus, the optimal time for cell/polymer harvest will vary based on the fermenting organism and the method of fermentation (shake-flask or fermenter).

Compositional analysis and characterization of the PHA polymers can been accomplished through ¹H and ¹³C-NMR solution spectroscopy, in order to quantify the molar ratio of HV/HB for various amounts and ratios of xylose and levulinic acid and otherwise to optimize fermentation conditions.

### EXAMPLES

### Example 1

**Microroganism and Fermentation conditions.** Shake flask experiments employing *Burkholderia cepacia* (formerly *Pseudomonas cepacia*) ATCC 17759 were carried out using 585 ml cultures composed of: 500 ml nitrogen-limited, defined mineral salt/trace element solution as described by Bertrand *et al*. (1990), 33 ml of concentrated xylose solution (from 36 % w/v stock, 2.2 % w/v final broth concentration), 2 ml of levulinic acid solution (from 42 % w/v stock solution in distilled water, pH adjusted to 7.2 with NaOH, 0.07 % w/v final broth concentration), and 50 ml of a seed culture inoculum. Nitrogen was further limited by reducing the (NH₄)₂SO₄ concentration to 1.5 g/L. The preinoculum seed cultures for all experiments were prepared in 2800 ml Fernbach flasks containing 0.8% w/v nutrient broth or 2.2 % w/v xylose and 0.07 % w/v levulinic acid, incubated at 28°C and 150 rpm for 72 hours. PHA production cultures were incubated at 28°C and 150 rpm for 20 hours (OD₅₄₀: 0.3 -0.5 ), at which time second doses of the above described levulinic acid solution were added to the appropriate cultures. Shake-flasks were incubated for another 48-50 hours and harvested (OD₅₄₀ : 2.5 - 3.0) for biomass and PHA extraction.

**Biomass and PHA sample preparation.** Broth cultures were harvested from shake flasks by centrifugation in 250 ml Nalgene bottles at 10,000 rpm for 10 minutes. Wet cell pellets were then washed in 100 ml dH₂0, subjected to a second centrifugation, and transferred to 200 ml glass bottles for lyophilization (-50°C, 0.05 mmHg vacuum pressure, 12-18 hours). Dried cell pellets were weighed, ground to powder, suspended in chloroform (*i*.*e*. 0.1 g lyophilized cell powder/ml), and incubated in 150 ml glass jars at 60°C for 24 hours. [P(3HB-co-3HV)] co-polymer was precipitated from the viscous chloroform solutions by 1:10 mixture with 95% EtOH and subsequently filtered through Whatman #1, 9 cm paper. Dried, white [P(3HB-co-3HV)] co-polymer cakes were resolubilized in chloroform for secondary and tertiary debris extractions, before being solvent-cast into 10 cm Pyrex petri dishes for film sample preparation. Residual chloroform solvent was removed from the [P(3HB-co-3HV)] co-polymer film samples by vacuum oven drying at 70°C and 20 in Hg vacuum pressure for 24 hours. The tested films were solvent cast into films 60-100 days following their initial production and aged at room temperature for approximately 60 days prior to physical/thermal characterization.

### Example 2

*B*. *cepacia* ATCC 17759 was grown essentially as described in Example 1 except that levulinic acid was re-fed at 20 hours and the amount of levulinic acid in the re-feeding was varied in different batches. Specifically, levulinic acid was added to all cultures initially as a 0.07 % w/v dose and then as progressively increasing doses (generally 0.1 to 0.6 % w/v) at 20 hours post-inoculation. Thus, the ratio of levulinic acid to xylose in the final fermentation medium varied from 0.03, i.e., 2.2:0.07, (no second dose of levulinic acid) to 0.3, i.e., 2.2:0.67 (second levulinic acid dose of 0.6 % w/v).

Compositional characterization of these PHA samples through 300 MHz ¹H NMR, ¹³C NMR, and X-ray diffraction studies has determined the solvent-cast films to be co-polymers composed of 3-hydroxybutyrate and 3-hydroxyvalerate (P(3HB-co-3HV). The table below shows the effects of differing amounts of levulinic acid in the second feeding on the relative amount of 3HV in the final P(3HB-co3HV) co-polymer. The amount of levulinic acid in the second feeding had no substantial effect on total biomass collected or the yield of co-polymer.

| Amount of Levulinic acid (% w/v) | Biomass (g/L) | P(3HB-co-3HV) (g/L) | mol% HV | PHA (% of biomass) | pH at harvest | OD540 at harvest |
|---|---|---|---|---|---|---|
| 0 | 5.35 | 2.75 | 0 | 51 | 6.58 | 2.992 |
| 0.25 | 5.57 | 2.8 | 10 | 50 | 7 | 2.859 |
| 0.35 | 5.85 | 3.1 | 18 | 53 | 7.38 | 2.904 |
| 0.45 | 5.54 | 2.72 | 22 | 49 | 7.69 | 2.882 |
| 0.5 | 5.32 | 2.67 | 26 | 50 | 7.82 | 2.851 |
| 0.55 | 5.26 | 2.62 | 30 | 50 | 7.81 | 2.841 |

### References

Aldor and Keasling (2001). Metabolic engineering of poly(3-hydroxybutyrate-co-3-hydroxyvalerate) composition in recombinant Salmonella enterica Serovar Typhimurium. Biotechnol. And Bioeng 76:108-114.
Bertrand, J.L., B. A. Ramsay, J. A. Ramsay, and C. Chavarie. 1990. Biosynthesis of poly-β- hydroxyalkanoates from pentoses by Pseudomonas pseudoflava. Appl. Environ. Microbiol. 56:3133-3138.
Bozell, J. J., I. Moens, D. C. Elliott, Y. Wang, G. G. Neuenschwander, S. W. Fitzpatrick, R. J. Bilski, J. L. Jarnefeld. 2000. Production of levulinic acid and use as a platform chemical for derived products. Resources, Conservation and Recycling 28:227-239.
Byrom, D. 1987. Polymer syntheseis by microorganisms: technology and economics. Trends Biotechnol. 5:246-250.
Choi et al., 1998, Cloning of the Alcaligenes latus polyhydroxyalkanoate biosynthesis genes and use of these genes for enhanced production of poly(3-hydroxybutyrate) in Escherichia coli, Appl. Environ. Microbiol. 64, 4897-4903.
Choi, J., S.Y. Lee. 1999. High-Level Production of Poly(3-Hydroxybuyrate-co-3-Hydroxyvalerate) by Fed-Batch Culture of Recombinant Esherichia coli. Appl. Environ. Microbiol. 65:4363-4368.
Doi, Y. 1990. Microbial polyesters. VCH Publishers, Inc., Yokohama, Japan.
Holmes, P.A. 1988. Biologically produced PHA polymers and co-polymers, p.1-65. In D.C. Bassett (ed.), Developments in crystalline polymers, vol. 2. Elsevier, London, United Kingdom.
   Horowitz et al., U.S. 6,368,836, 2002.
Imam, S. H., S. H. Gordon, R. L. Shogren, and T.R. Tosteson, N.S. Govind, and R.V. Greene. 1999. Degradation of starch-poly(β-hydroxybuyrate-co-β-hydroxyvalerate) bioplastic in tropical coastal waters. Appl. Environ. Microbiol. 65:431-437.
Jang, J.H., and P.L. Rogers. 1996. Effect of levulinic acid on cell growth and poly-β-hydroxyalkanoate production by Alcaligenes sp. SH-69. Biotechnol. Lett. 18:219-224.
Kim, B.S., S.C. Lee, S.Y. Lee, H.N. Chang, Y.K. Chang, S.I. Woo. 1994. Production of poly(3-hydroxybutyric-co-3-hydroxyvaleric acid) by fed-batch culture of Alcaligenes eutrophus withsubstrate control using on-line glucose analyzer. Enzyme Microb. Technol. 16:556-561.
Madison, L.L., G.W. Huisman. 1999. Metabolic Engineering of Poly(3-hydroxyalkanoates): From DNA to Plastic. Microbiol. and Molec. Biol. Reviews. 63:21-53.
Naylor et al., U.S. 5,871,980
Ramsay, J., M. Hassan, and B. Ramsay. 1995. Hemicellulose as a potential substrate for production of poly(β-hydroxyalkanoates). Can. J. Microbiol. 41:262-266.
Schubert et al., 1998, Cloning of the Alcaligenes eutrophus poly- b-hydroxybutyrate synthetic pathway and synthesis of PHB in Escherichia coli. J. Bacteriol. 170, 5837-5847.
Steinbuchel, A., G. Schmack, V. Gorenflo. 1998. Biotechnological production and characterization of polyesters containing 4-hydroxyvaleric acid and medium-chain-length-hydroxyalkanoic acids. Macromolecules 31:644-649.
Sudesh, K., H. Abe, Y. Doi. 2000. Synthesis, structure, and properties of polyhydroxyalkanoates: biological polyesters. Prog. Pol. Sci. 25:1503- 1555.
Chung, S.H., Choi, G.G., Kim, H.W., Rhee, Y.H. 2001. Effect of Levulinic Acid on the Production of Poly(3-hydroxybutyrate-co-3-hydroxyvalerate) by Ralstonia eutropha KHB-8862. J. Microbiol. vol 39, no. 1, March 2001: 79-82.

## Claims

1. A process for producing polyhydroxyalkanoates (PHA) co-polymer comprising 3-OH-valeryl (3-HV) and 3-OH-butyryl (3-HB) monomers, the process comprising: adding to a medium containing *Burkholderia cepacia* a quantity of xylose as a primary carbon source and a first quantity of levulinic acid as a secondary carbon source; adding a second quantity of levulinic acid to the medium between about 16 hours and about 24 hours after the first quantity of levulinic acid was added,
wherein the second quantity of levulinic acid is greater than the first quantity of levulinic acid.

2. The process of claim 1, wherein the second quantity of levulinic acid is added about 20 hours after the first quantity of levulinic acid was added.

3. The process of claim 1, wherein the first quantity of levulinic acid is about 0.07 % w/v and the second quantity of levulinic acid is about 0.1 to 0.8 % w/v.

4. The process of claim 1 in which the xylose is derived from the xylans present in hemicellulose.

5. The process of claim 4 in which the hemicellulose is derived from forest biomass.

6. The process of claim 4 in which the levulinic acid is derived from organic waste.

7. The process of claim 5 in which the levulinic acid is derived from forest biomass.

8. The process of claim 1 in which the ratio of HV to HB is modulated by adjusting the ratio of xylose to levulinic acid.

9. The process of claim 1 in which the ratio of xylose to levulinic acid in the fermentation medium after the additional amounts of levulinic acid are added ranges from about 0.01 to about 1.0.

10. The process of claim 1 in which the xylose is oxidized or otherwise derivatized prior to or after addition to the culture medium.

11. The process of claim 1 in which the levulinic acid is oxidized or otherwise derivatized prior to or after addition to the culture medium.

## Patentansprüche

1. Verfahren zur Herstellung eines Polyhydroxyalkanoat- (PHA) Copolymers mit 3-OH-Valeryl- (3-HV) und 3-OH-Butyryl- (3-HB) Monomeren, wobei das Verfahren umfasst:
Zugeben einer Menge an Xylose als primäre Kohlenstoffquelle und einer ersten Menge an Lävulinsäure als sekundäre Kohlenstoffquelle zu einem Medium, das Burkholderia cepacia enthält;
Zugeben einer zweiten Menge an Lävulinsäure zum Medium zwischen etwa 16 Stunden und etwa 24 Stunden, nachdem die erste Menge an Lävulinsäure zugegeben wurde,
wobei die zweite Menge an Lävulinsäure größer ist als die erste Menge an Lävulinsäure.

2. Verfahren nach Anspruch 1, wobei die zweite Menge an Lävulinsäure etwa 20 Stunden, nachdem die erste Menge an Lävulinsäure zugegeben wurde, zugegeben wird.

3. Verfahren nach Anspruch 1, wobei die erste Menge an Lävulinsäure etwa 0,07 % Gewicht/Volumen ist und die zweite Menge an Lävulinsäure etwa 0,1 bis 0,8 % Gewicht/Volumen ist.

4. Verfahren nach Anspruch 1, wobei die Xylose von den Xylanen, die in Hemicellulose vorhanden sind, abgeleitet ist.

5. Verfahren nach Anspruch 4, wobei die Hemicellulose von forstlicher Biomasse abgeleitet ist.

6. Verfahren nach Anspruch 4, wobei die Lävulinsäure von organischem Abfall abgeleitet ist.

7. Verfahren nach Anspruch 5, wobei die Lävulinsäure von forstlicher Biomasse abgeleitet ist.

8. Verfahren nach Anspruch 1, wobei das Verhältnis von HV zu HB durch Einstellen des Verhältnisses von Xylose zu Lävulinsäure moduliert wird.

9. Verfahren nach Anspruch 1, wobei das Verhältnis von Xylose zu Lävulinsäure im Fermentationsmedium, nachdem die zusätzlichen Mengen an Lävulinsäure zugegeben sind, im Bereich von etwa 0,01 bis etwa 1,0 liegt.

10. Verfahren nach Anspruch 1, wobei die Xylose vor oder nach der Zugabe zum Kulturmedium oxidiert oder anderweitig derivatisiert wird.

11. Verfahren nach Anspruch 1, wobei die Lävulinsäure vor oder nach der Zugabe zum Kulturmedium oxidiert oder anderweitig derivatisiert wird.

## Revendications

1. Processus destiné à produire un copolymère de polyhydroxyalkanoates (PHA), comprenant des monomères 3-OH-valéryl (3-HV) et 3-OH-butyryl (3-HB), le processus comprenant les opérations consistant à :
ajouter à un milieu, contenant des *Burkholderia cepacia*, une certaine quantité de xylose en tant que source de carbone primaire et une première quantité d'acide lévulique en tant que source de carbone secondaire ;
ajouter une seconde quantité d'acide lévulique au milieu entre environ 16 heures et environ 24 heures après que la première quantité d'acide lévulique a été ajoutée ;
dans lequel la seconde quantité d'acide lévulique est supérieure à la première quantité d'acide lévulique.

2. Processus selon la revendication 1, dans lequel la seconde quantité d'acide lévulique est ajoutée environ 20 heures après que la première quantité d'acide lévulique a été ajoutée.

3. Processus selon la revendication 1, dans lequel la première quantité d'acide lévulique est d'environ 0,07 % poids/volume et la seconde quantité d'acide lévulique est d'environ 0,1 à 0,8 % poids/volume.

4. Processus selon la revendication 1, dans lequel le xylose est tiré des xylanes, présents dans l'hémicellulose.

5. Processus selon la revendication 4, dans lequel l'hémicellulose est tirée de la biomasse forestière.

6. Processus selon la revendication 4, dans laquelle l'acide lévulique est tiré de déchets organiques.

7. Processus selon la revendication 5, dans lequel l'acide lévulique est tiré de la biomasse forestière.

8. Processus selon la revendication 1, dans lequel le rapport HV à HB est modulé en ajustant la proportion de xylose par rapport à l'acide lévulique.

9. Processus selon la revendication 1, dans lequel la proportion de xylose par rapport à l'acide lévulique dans le milieu de fermentation, après que les quantités supplémentaires d'acide lévulique ont été ajoutées, varie d'environ 0,01 à environ 1,0.

10. Processus selon la revendication 1, dans lequel le xylose est oxydé ou dérivé d'une autre manière avant ou après addition au milieu de culture.

11. Processus selon la revendication 1, dans lequel l'acide lévulique est oxydé ou dérivé d'une autre manière avant ou après addition au milieu de culture.
